(19) Europäisches Patentamt / European Patent Office / Office européen des brevets

(11) **EP 4 654 134 A1**

(12) **EUROPEAN PATENT APPLICATION**

(43) Date of publication:
**26.11.2025 Bulletin 2025/48**

(21) Application number: **24177645.9**

(22) Date of filing: **23.05.2024**

(51) International Patent Classification (IPC):
*G06T 11/00* (2006.01)

(52) Cooperative Patent Classification (CPC):
**G06T 11/005;** G06T 2211/444; G06T 2211/448

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• Siemens Healthineers AG
**91301 Forchheim (DE)**

• Friedrich-Alexander-Universität Erlangen-Nürnberg
**91054 Erlangen (DE)**

(72) Inventors:
• **Kreher, Björn**
**91094 Bräuningshof (DE)**
• **Rohleder, Maximilian**
**91052 Erlangen (DE)**

(74) Representative: **Siemens Healthineers Patent Attorneys**
**Postfach 22 16 34**
**80506 München (DE)**

(54) **PREDICTING ARTEFACTS IN 3D IMAGING**

(57) The invention relates to a method of estimating artifacts in 3D imaging by providing a 3D mask (14) representing an object. X-rays of an X-ray source-detector pair (2, 3) are simulated through the object in a plurality of projection positions of the X-ray source-detector pair (2, 3) moving along a pregiven trajectory. An artifact value is assigned to each voxel of a 3D artifact image (19) depending on respective path lengths of the X-rays through the 3D mask. Visualizing a respective artifact map for a current C-arm tilt enables an interactive optimization of a C-arm tilt.

FIG 2

EP 4 654 134 A1

## Description

The present invention relates to a method of estimating artifacts in 3D imaging including the step of providing a 3D mask representing an object. Furthermore, the present invention relates to an X-ray device.

Independent of the grammatical term usage, individuals with male, female or other gender identities are included within the term.

Mobile C-arm systems are used for guidance using 2D imaging during orthopedic and trauma procedures and are increasingly used for 3D verification of implant placement using cone-beam computed tomography (CBCT) capabilities. Especially in treatment of spinal fractures the intraoperative validation of correct implant positioning using 3D imaging is crucial, as the anatomies of interest and placement of screws within them is hard to verify on projection images. In this context, metal artifacts emerging in and around metallic objects in the CBCT reconstruction obstruct clinically relevant image features and therefore harden clinical decision making.

As metal artifacts in CBCT images alter the shape of metallic implants and obscure the depicted anatomy surrounding these objects (e.g. a bone around the shaft of a screw is hidden by metal artifacts), the imaging modality's usefulness for intraoperative validation is drastically reduced.

So-called metal artifact reduction (MAR) methods in postprocessing, fail when the artifacts are significant. Inpainting-based methods (e.g. FS-MAR Frequency Split metal artifact reduction; https://pubmed.ncbi.nlm.nih.gov/22482612/) implemented in the product), first segment metal voxels in a initial reconstruction, then process the projection data in the projection-trace of the segmentation to remove the contribution of metal to the reconstruction. In cases of strong artifacts, the deformation of metallic objects leads to over-/under segmentation of the object, which in turn negatively impacted the algorithms performance.

Another class of methods, called metal artifact avoidance (MAA), seeks to improve the quality of the acquired raw data by adaptation of the source-detector trajectory during image acquisition. By avoiding projection images with a large metal-bias, the image reconstructed from this higher-fidelity data exhibits less artifacts. As the optimized trajectory depends on the position, shape and orientation of the metal objects, additional X-ray images, so called scout views, are needed to predict an artifact avoiding trajectory for the scene to be imaged. The trajectory resulting from this optimization can either be circular or non-circular. While literature reports superior performance of non-circular orbits, circular orbits are easier to realize due to regulatory and practical reasons.

An approach of Wu, P., Sheth, N., Sisniega, A., Uneri, A., Han, R., Vijayan, R., Vagdargi, P., Kreher, B., Kunze, H., Kleinszig, G., Vogt, S., Lo, S.F., Theodore, N., Siewerdsen, J.H.: C-arm orbits for metal artifact avoidance (MAA) in cone-beam CT; Physics in Medicine and Biology 65 (16) (8 2020). https://doi.org/10.1088/1361-6560/ab9454 investigates the feasibility of optimizing a circular or non-circular trajectory given a few scout view X-ray images.

Rohleder, M., Kunze, H., Kleinszig, G., Maier, A., Kreher, B.: Robust 3D Metal Segmentation from X-Ray Projection Images for Metal Artifact Trajectory Optimization. In: Medical Imaging 2024: Physics of Medical Imaging (2024) https://doi.org/10.1117/12.3005260 show an end-to-end deep learning approach backprojection and subsequent segmentation of metallic objects.

Siddon, R.L.: Fast calculation of the exact radiological path for a three-dimensional CT array. Medical Physics 12 (2), 252-255 (3 1985). https://doi.org/10.1118/1.595715 introduces a raytracing forward projection for a given C-arm tilt.

US 11 790 525 B2 introduces a concept of Metal Artifact Avoidance (MAA). This patent uses trajectory scores in the form of an objective function which is a single score per trajectory and thus a so called Global-MAA.

The object of the present invention is to predict the metal artifacts more accurately.

According to the present invention, this object is solved by a method and an X-ray device according to the independent claims. Furthermore, a respective computer program and a computer-readable medium are provided.

Advantageous further developments result from the subclaims.

Specifically, there is provided a method of estimating artifacts in 3D imaging. Artifacts occur in 3D imaging, when e.g. a patient is examined by X-ray imaging, and the patient has screws or other metal objects implanted in a part of his body (volume of interest). Metal artifacts hide anatomical structures around the metal objects. For instance, a bone region into which a metal screw is screwed is hidden by the metal artifacts and is not shown in the X-ray image. Other objects, such as bone structures, can also cause artifacts. Such artefacts shall be estimated or predicted by the present method.

In one step of the inventive method there is provided at least one 3D mask representing the object (When we refer to a mask in the following, we may also mean several masks.). The object may be one of a plurality of objects. Furthermore, the object may consist of a plurality of single parts. For example, there are several screws in the spine of a patient. One of the screws or the total arrangement of the screws may be regarded as the metal object. The 3D mask may contain a voxel or parametric description of the object.

In a further step of the inventive method, X-rays of an X-ray source-detector pair of an X-ray device through the object are simulated in a plurality of projection positions of the X-ray source-detector pair moving along a trajectory. For example, the source-detector pair of a C-arm system is moved along a circular orbit (trajectory) by the C-arm. However, the source-detector pair can also move along another trajectory which differs from a circular shape. The source-detector pair takes up a certain protection position on the trajectory. In the case of the C-arm system, the X-ray source and X-ray detector are mounted at the ends of the C-arm and stand opposite each other in the projection positions. Respective X-rays in the different projection positions of the X-ray source-detector pair are simulated when passing through the object or object model.

A further step of the invention method includes assigning an artifact value to each voxel of a 3D artifact image depending on respective path lengths of the X-rays through the 3D mask. The assignment can be performed by any function or relation (e.g. linear function, quadratic function, look up table etc.).

In one embodiment the step of assigning the artifact value to each voxel of the 3D artifact image additionally depends on one or more further parameters which are provided for or by an X-ray system, the X-ray source-detector pair belongs to. Thus, specific parameters characterizing the actual examination situation can be regarded when assigning artifact values to the voxels.

According to another embodiment at least one of the further parameters comprises meta data of the X-ray system, navigation data for navigating the object between the X-ray source-detector pair or geometric data of the object. For instance, the meta data may include controlling data of the X-ray system. The navigation data may include position data of the object within the X-ray system. The geometric data may relate to the corpulence of a patient.

In a specific embodiment the method includes a step of determining a respective 2D path length projection for each projection position, wherein each 2D path length projection includes values each representing a respective path length of one of the simulated X-rays through the object. Thus, the simulated X-rays of each of the projection positions are used to generate a respective 2D projection for the respective projection position. The 2D path length projections are obtained by calculating line integrals along the simulated X-rays, thereby accumulating the track or path length in the object. For example, one pixel of the 2D path length projection is dark, if the simulated X-ray has a long track length through the object. Otherwise, a pixel of the 2D path length projection is white, if an X-ray does not travel through object.

In a further step, a respective artifact value may be assigned to each path length, thereby obtaining a set of 2D artifact projections from the 2D path length projections. This means, that the 2D path length projections obtained from the step above are transformed into 2D artifact projections. The transformation is performed by assigning a corresponding artefact value to each path length value. After transformation, each pixel of the 2D artifact projection represents an artifact value. Thus, an absolute artifact strength is available for each local position. Consequently, the 2D artifact projections provide a local resolution of absolute artifact strengths.

Finally, a back projection may be performed on the set of 2D artifact projections in order to obtain a 3D artifact image. Thus, each voxel of the 3D artifact image represents an absolute artifact strength. In other words, the 3D artifact image represents the object with artifact strength values. Thus, the 3D artifact image shows a volumetric resolution of the artifacts generated by the object.

The advantage of the inventive method is that there is not computed a single score per scene, which does not allow to localize the expected artifacts. According to the inventive method, there is rather computed where in the 3D scene artifacts are to be expected after image acquisition. This allows to condition the trajectory optimization on a region of interest, and further allows the user to control image quality at the object-level.

In a specific embodiment, the one or more 3D masks are obtained by reconstructing 2D X-ray scout views. Usually, such scout views are gained for the orientation of the physician. The scout views are typically obtained at lower doses. The 3D mask(s) may be generated by back projection.

According to another embodiment, the 3D mask represents the object and at least one additional object, and the 3D mask is obtained based on segmenting the objects. This means that at least two objects are present in a volume of interest. For instance, there are two or more screws in a spine region to be examined. The metal objects are segmented in order to separate them reliably. For such segmentation, usual segmentation algorithms can be used.

According to another embodiment, the trajectory has a main plane being tilt in a pregiven coordinate system. For instance, the trajectory is a circular orbit having a main plane perpendicular to the center axis of the orbit. The main plane can be tilt at a certain angle (tilt angle). This tilt angle can be zero or may have a positive or negative value. Typical tilt angles lie in the range of -30 to 30 degrees. Often such a tilt leads to an improved X-ray image.

In a further embodiment, the 3D artifact image is projected forward onto one of the scout views to obtain an overlay image. Thus, the artifacts are integrated into the scout views so that the physician can use the familiar scout views enriched with artifact information.

In a further embodiment, an artifact strength is color-coded in the 2D artifact projections or the 3D artifact image. The color-coding makes it easier to recognize the quality of the X-ray image. For instance, red pixels or voxels indicate regions with strong artifacts, whereas blue pixels or voxels indicate minor artifacts. In this case, a user can simply recognize and localize regions with strong artifacts in the volume of interest.

Specifically, the colors used for the color-coding may be calibrated by determining a specific color for an artifact threshold of a reconstructed calibration phantom (e.g. metal wedge). The metal length through a metal wedge increases along one dimension. Thus, a specific metal length can be chosen in the X-ray image which leads to still acceptable artifacts. A corresponding color (e.g. red) can be assigned to this threshold length. Such calibration leads to color-coded artifacts which can be compared with each other. Consequently, the calibrated color-coding allows to predict absolute artifact strengths. While previous methods just predict one score for one trajectory and only allow relative comparison of different trajectories, the inventive method can indicate if a trajectory will result in good or bad image quality. This is based on the calibration, which makes voxel impacts comparable over different scenes. This allows the user to decide if a tilt of a C-arm, for example, is necessary at all.

Additionally, there is provided a method of adjusting an X-ray device by estimating artifacts as described above and adjusting the tilt of the trajectory of the X-ray source-detector pair in dependence of the 3D artifact image or a forward projection of the 3D artifact image. The adjustment of the tilt of the trajectory of the X-ray source-detector pair allows for optimizing the path of the X-rays through the object. Typically, shorter paths through the object lead to less artifacts. Thus, by tilting the trajectory in an appropriate manner, the artifacts can be reduced or optimized.

In a specific embodiment, the adjustment is performed automatically by minimizing artifact values of the 3D artifact image or a forward projection of the 3D artifact image by varying the tilt of the X-ray source-detector pair. Specifically, the artifacts in a specific region of interest shall be reduced under a certain threshold. This can be achieved by varying the tilt of the trajectory main plane (i.e. the plane of the X-ray source-detector pair) until the artifact values are below the certain threshold. Such an adjustment can be performed automatically but also manually. The manual adjustment requires an interactive control of the X-ray device for X-ray source-detector pair.

Moreover, in another embodiment the artifact values of a region of the 3D artifact image or the forward projection of the 3D artifact image are minimized, and the region is specified by a task. Thus, a task specific region is optimized with regard to artifacts. For instance, the task can be aimed at optimally imaging a small group of screws. In this case, this group of screws can be minimized, and artifacts of screws out of this region are irrelevant.

As indicated above, the adjustment may also be performed interactively by an operator of the X-ray source-detector pair, and the 3D artifact image or a forward projection of the 3D artifact image may be updated when the tilt is varied. Thus, the operator can easily recognize whether a specific variation of the tilt leads to a better result or not.

As indicated above, the object may be a metal object or at least a part of a human or animal body. For example, the object is a bone structure of a shoulder or a skull. The 3D mask can be regarded as a model of the object.

The above object is also solved by an X-ray device including an X-ray source-detector pair and estimating means capable of performing the above-described method. The estimating means may include a computer or processor for calculating the respective metal artifacts.

The advantages and further developments of the method described above apply analogously to the X-ray device. Thus, the described method steps can be regarded as functional features of the X-ray device.

In a specific embodiment, the X-ray source-detector pair of the X-ray device is fixed at a C-arm. Thus, the X-ray device represents a C-arm system.

Additionally, there may be provided a computer program comprising instructions which, when the program is executed by a computer or an X-ray device mentioned above, cause the computer or X-ray device to carry out the method defined above.

Moreover, there may be provided a computer-readable medium comprising instructions which, when executed by a computer or an X-ray device above, cause the computer or X-ray device to carry out the method defined above.

The present invention will now be described in more detail in connection with drawings showing in:

FIG 1    a principal arrangement of a C-arm system;

FIG 2    a block diagram for localization of predicted metal artifacts and interactive circular orbit optimization work flow;

FIG 3    a C-arm with respective coordinates;

FIG 4    a phantom for calibrating a CBCT system;

FIG 5    an X-ray image of the phantom; and

FIG 6    a colorized voxel impact map.

The following embodiments represent preferred examples of the present invention.

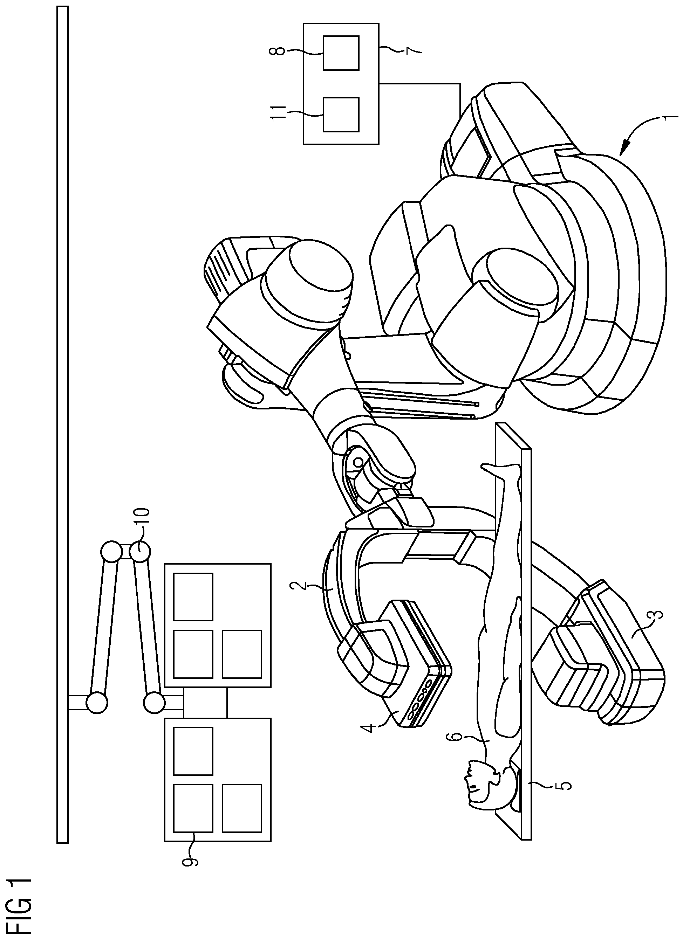

FIG 1 shows an example of a monoplane X-ray system with a C-arm 2 held by a stand 1 in the form of a six-axis industrial or articulated robot, at the ends of which an X-ray radiation source, for example an X-ray source 3 with X-ray tube and collimator, and an X-ray image detector 4 are attached as an image acquisition unit. The realization of the X-ray diagnostic device is not dependent on the industrial robot. Conventional C-arm devices can also be used.

A patient 6 or a technical object to be examined is positioned on a table top 5 of a positioning table in the beam path of the X-ray emitter 3. A system control unit 7 with a computer 8 for image processing is connected to the X-ray diagnostic device, which receives and processes the image signals from the X-ray image detector 4 (control elements are not shown, for example). The X-ray images can then be viewed on the displays of a monitor lamp 9. The monitor lamp 9 can be held by means of a ceiling-mounted, longitudinally movable, pivotable, rotatable and height-adjustable support system 10 with a cantilever and lowerable support arm. An estimating system 11 for estimating metal artifacts in 3D imaging is also provided in the system control unit 7.

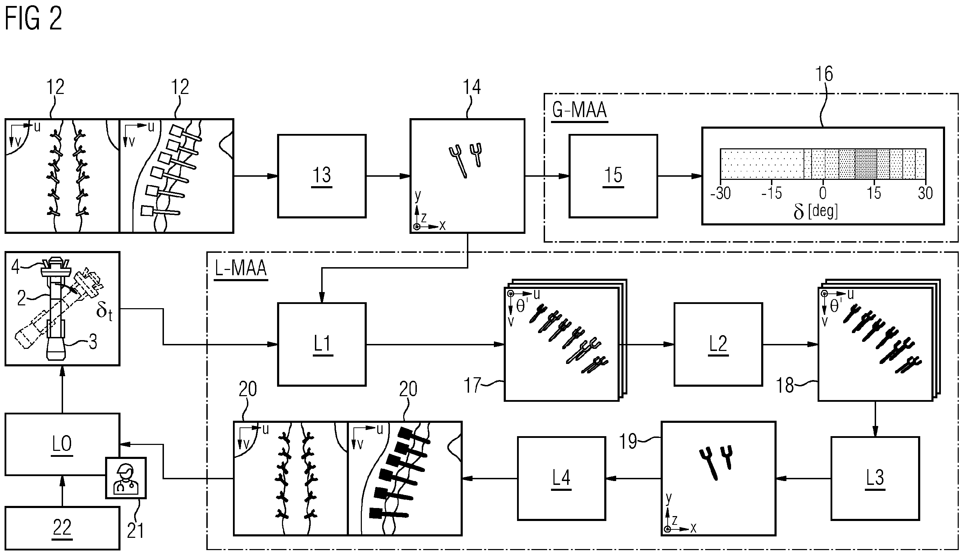

The examples of FIGs 2 to 6 relate to metal objects. These metal objects are used representatively for all possible objects (e.g. bones etc.).

A processing pipeline shown in FIG 2 may be employed to calculate Global-MAA scores (G-MAA) and/or spatially resolved Local-MAA (L-MAA) overlays for a current C-arm tilt $\delta_t$ (bottom). Given two or more scout views 12 of the scene, (metal, bone, etc.) distribution can be estimated as a volumetric (meta, bone, etc.)l mask 14 $b_{seg}$ (x, y, z). This can be done either by backprojection and subsequent segmentation 13 of metallic objects as detailed in Wu et al. or using a end-to-end deep learning approach as described in Rohleder et al. This component of the pipeline can be assumed given.

From this knowledge of metal distribution, a score 16 is derived for each trajectory in a computation step 15 following e.g. the Qpoly objective function from Wu et al. Projection images may be computed for each tilted circular scan and the mean spectral shift per simulated projection can be computed. For instance, a trajectory is scored by computing the variance over the averaged spectral shift values of its constituting projection images. The resulting 1D objective function Qpoly ($\delta$) is normalized relative to the worst and best possible trajectory over the evaluated angular range from $\delta \in [$ -30 , 30] degrees as exemplary shown by trajectory score 16. Details for this procedure can be found in Wu et al.

To compute the spatial distribution of expected metal artifacts (Local-MAA or L-MAA in FIG 1) we follow the inventive approach. Initially, the current tilt $\delta_t$ of C-arm 2 is read out and pathlength images p$\delta_t$ ( u, v, $\theta$ ) are computed as line integrals through the binary metal volume bseg (x, y, z). I.e. X-ray paths through the metal are simulated in a step L1. A respective set of line integral projections 17 (herein also called 2D metal length projections) is obtained.

Defining a system matrix $A_{\theta,\delta t}$ as the Siddon (see above) raytracing forward projection for a given C-arm tilt $\delta_t$ and rotation $\theta$, this can be expressed as

$$p_{\delta t} ( u, v, \theta ) = A_{\theta,\delta t} b_{seg} .$$

To better model the artifact bias induced by a certain projection length of metal, we compute the spectral shift defined as the difference between the monoenergetic and polyenergetic forward model similar to Wu et al. Thereby an respective artifact strength value is assigned to each metal length value pixel by pixel (step L2) to obtain a set of metal artifact projections 18 (herein also called 2D artifact projections). However, in contrast to the Global-MAA computation, we compute this per pixel instead of summing over each projection image.

In step L3, the volumetric impact per unit volume of these projection domain spectral shift maps is calculated. For each voxel defined as metal in the metal mask 14 ($b_{seg}$) a value may be computed as the variance over its projected locations' values in the previously computed spectral shift images, i.e. the metal artifact projections 18, thereby obtaining a 3D artifact image 19 (voxel impact). While this seems similar to the objective function $Q_{poly}$ (trajectory score 16) the notable difference here is that this value is normalized over a unit volume and is thus an absolute measure for the expected artifact strength at this volumetric location. This results in the voxel impact maps $m_{\delta t}$ (x, y, z) which can be expressed as

$$m_{\delta t} ( x, y, z ) = Var_\theta ( s_{\delta t} ( u*, v*, \theta )),$$

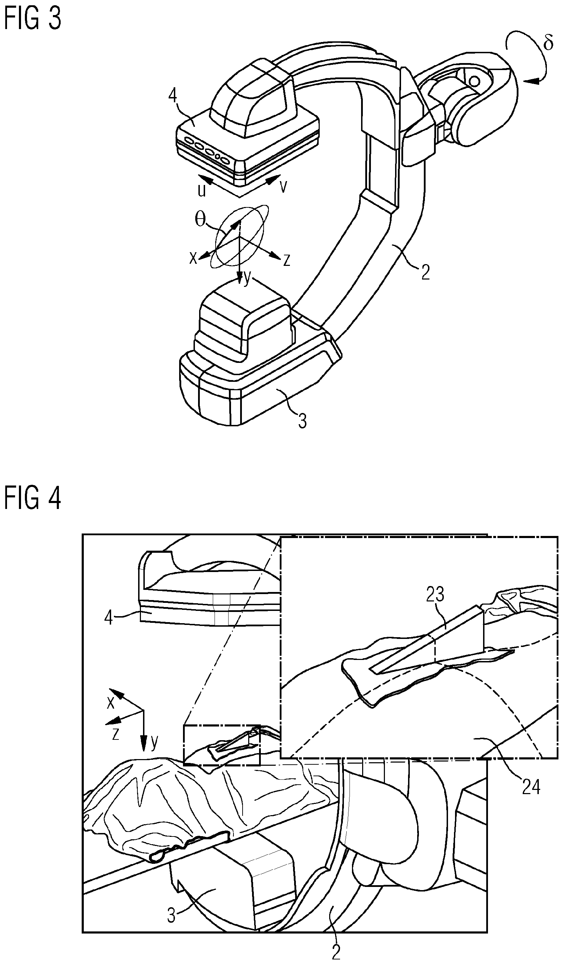

where u* and v* are the detector coordinates of position (x, y, z) after forward projection into view $\theta$ under tilt angle $\delta t$ (compare FIG 3).

Finally, in step L4 one or more overlay images 20 (also called guidande overlays) are computed by e.g. maximum projection of the voxel impact map (3D artifact image 19) onto at least one of the scout views 12 along the known projection geometry. Maximum projection means that a maximum artifact value of all voxels contributing to a pixel of the 2D projection is used for the projection.

Based on this one or more guidance overlays 20, the user 21 can interactively choose (step L0) a trajectory by tilting the C-arm 2 to minimize the displayed artifact prediction. In this way the C-arm tilt can be optimized interactively with respect to a clinical task 22 (e.g. watching two specific screws in a spine). Thus, the clinician is enabled to find an optimized scanning trajectory while factoring in an imaging task derived from clinical conditions and the procedural context.

In order to standardize the unbounded and initially incomprehensible values derived from the variance of spectral shift per voxel unit-volume, a basic calibration method is provided as shown in FIGs 4 to 6. The objective is to establish a metric value that represents a significant degree of artifacts and designate this value as the upper limit in the colormap used for presentation to the physician.

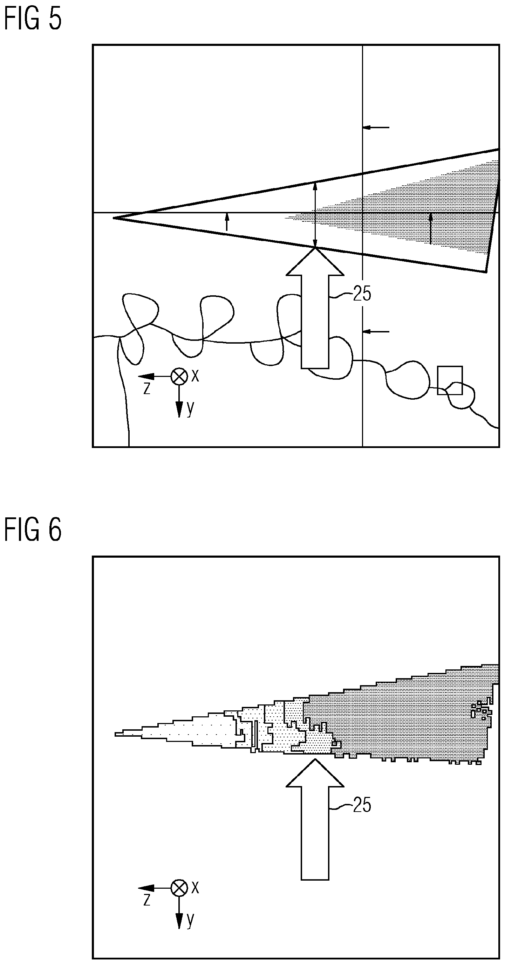

As illustrated in FIG 4 , a wedge phantom 23 (e.g. 50×150×5mm) made of titanium for instance is positioned along the z-axis of the C-arm gantry on top of a cadaver 24 for realistic patient contrast. After acquisition of a CBCT scan, the volume shown in FIG 5 is inspected in slices through the wedge phantom for empirically identifying a "critical artifact threshold". In the determined slice (see arrow 25), the height of the wedge phantom was measured as 25mm. This may be done by a trained orthopedic surgeon by evaluating the homogeneity of intensity within the metallic object and the severity of streaking artifacts surrounding the wedge phantom. Using a threshold based segmentation of the object, steps L1-L3 of the previously described method are evaluated to yield a respective voxel impact map (3D artifact image) of the wedge phantom 23. The mean value at the empirically determined height (position of arrow 25) is read out. This mean or average value is used to define the color mapping illustrated in FIG 6. Henceforth this mean value can be used to normalize the computed voxel impact for color hue mapping in the final overlay images 20.

The above exemplary calibration protocol is chosen, as the phantom models a monotonous increase in variance of spectral shift. The thickness of the wedge is loosely similar to the diameter of pedicle screws (approx. 5mm) whilst the increasing height models increasing shaft lengths.

Based on the above described dynamically changing computed Local-MAA guidance an interactive optimization scheme can be provided using localized metal artifact predictions. As illustrated in FIG 2 , the clinician or user (operator) 21 interprets the displayed overlay 20 and adjusts the C-arm tilt, which in turn changes the shown overlay image 20 (guidance image). This interactive setting has several advantages. Making use of the clinical background knowledge and procedural context allows to condition the tilt-optimization problem and find a imaging-task specific optimum. Furthermore, should practical considerations such as obstructions in the operating room interfere with an optimal circular orbit, this can be accounted for and a next-best trajectory can be found as compromise. Finally, the absolute localized metrics may allow to judge if a tilt is necessary at all, which is not possible with Global-MAA scores as these are always normalized to best/worst case.

**Claims**

1. Method of estimating artifacts in 3D imaging by

   - providing a 3D mask (14) representing an object,
   **characterized by**
   - simulating X-rays of an X-ray source-detector pair (2, 3) through the object in a plurality of projection positions of the X-ray source-detector pair (2, 3) moving along a pregiven trajectory,
   - assigning an artifact value to each voxel of a 3D artifact image (19) depending on respective path lengths of the X-rays through the 3D mask.

2. Method according to claim 1, wherein the step of assigning includes:

   - determining a respective 2D path length projection (17) for each projection position, and wherein each 2D path length projection (17) includes values each representing a respective path length of one of the simulated X-rays through the 3D mask,
   - assigning a respective single artifact value to each path length, thereby obtaining a set of 2D artifact projections (18) from the 2D metal length projections (17) and
   - back projecting the set of 2D artifact projections (18) to the 3D artifact image (19).

3. Method according to claim 1 or 2, wherein the step of assigning the artifact value to each voxel of the 3D artifact image (19) depends on one or more further parameters which are provided for or by an X-ray system, the X-ray source-detector pair (2, 3) belongs to.

4. Method according to claim 3, wherein at least one of the further parameters comprises meta data of the X-ray system, navigation data for navigating the object between the X-ray source-detector pair (2, 3) or geometric data of the object.

5. Method according to one of the preceding claims, wherein the 3D mask (14) is obtained by reconstructing 2D X-ray scout views (12).

6. Method according to one of the preceding claims, wherein the 3D mask (14) represents the object and at least one additional object, and the 3D mask (14) is obtained based on segmenting the objects.

7. Method according to claim 5 or 6, wherein the 3D artifact image (19) is projected forward onto one of the scout views (12) to obtain an overlay image (20).

8. Method according to one of the preceding claims, wherein an artifact strength is color-coded in the 2D artifact projections (18) or the 3D artifact image (19).

9. Method according to claim 8, wherein colors used for the color-coding are calibrated by determining a specific color for an artifact threshold of a reconstructed calibration phantom (metal wedge) (23).

10. Method according to one of the preceding claims, wherein the trajectory has a main plane being tilt in a pregiven coordinate system.

11. Method of adjusting an X-ray device by estimating artifacts according to claim 10 and adjusting the tilt of the trajectory of the X-ray source-detector pair (2, 3) in dependence of the 3D artifact image (19) or a forward projection of the 3D artifact image (19).

12. Method according to claim 11, wherein the adjustment is performed automatically by minimizing artifact values of the 3D artifact image (19) or a forward projection of the 3D artifact image (19) by varying the tilt of the X-ray source-detector pair (2, 3).

13. Method according to claim 12, wherein the artifact values of a region of the 3D artifact image (19) or the forward projection of the 3D artifact image (19) are minimized, and the region is specified by a task.

14. Method according to claim 11, wherein the adjustment is performed interactively by an operator (21) of the X-ray source-detector pair (2, 3), and the 3D artifact image (19) or a forward projection of the 3D artifact image (19) is updated when the tilt is varied.

15. Method according to one of the preceding claims, wherein the object is a metal object or at least a part of a human or animal body.

16. X-ray device including an X-ray source-detector pair (2, 3) and estimating means capable of performing a method according to one of the preceding claims.

17. X-ray device according to claim 16, wherein the X-ray source-detector pair (2, 3) is fixed at a C-arm.

FIG 1

FIG 2

G-MAA

L-MAA

δ [deg]

-30   -15   0   15   30

FIG 3

FIG 4

FIG 5

FIG 6

| | Europäisches Patentamt European Patent Office Office européen des brevets | **EUROPEAN SEARCH REPORT** | **Application Number** EP 24 17 7645 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Wu Peiweng: "IMPROVED IMAGE QUALITY IN CONE-BEAM COMPUTED TOMOGRAPHY FOR IMAGE-GUIDED INTERVENTIONS", A dissertation submitted to Johns Hopkins University in conformity with the requirements for the degree of Doctor of Philosophy, 5 January 2022 (2022-01-05), XP093221927, Baltimore, Maryland Retrieved from the Internet: URL:http://jhir.library.jhu.edu/handle/1774.2/67199 [retrieved on 2024-11-07] * Chapter 4, "Metal Artifacts Avoidance (MAA) with Model-Driven Design of the Source-Detector Orbit" * ----- | 1-17 | INV. G06T11/00 |
| A,D | US 11 790 525 B2 (UNIV JOHNS HOPKINS [US]; SIEMENS HEALTHCARE GMBH [DE]) 17 October 2023 (2023-10-17) * column 11, line 32 - column 14, line 19 * ----- | 1-17 | TECHNICAL FIELDS SEARCHED (IPC) G06T |

The present search report has been drawn up for all claims

| Place of search | Date of completion of the search | Examiner |
|---|---|---|
| Munich | 7 November 2024 | Werling, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 7645

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2024

| Patent document cited in search report | | Publication date | Patent family member(s) | Publication date |
|---|---|---|---|---|
| US 11790525 | B2 | 17-10-2023 | CN 112927315 A | 08-06-2021 |
| | | | DE 102020131786 A1 | 10-06-2021 |
| | | | US 2021174502 A1 | 10-06-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11790525 B2

**Non-patent literature cited in the description**

- **WU, P.** ; **SHETH, N** ; **SISNIEGA, A** ; **UNERI, A.** ; **HAN, R.** ; **VIJAYAN, R** ; **VAGDARGI, P.** ; **KREHER, B** ; **KUNZE, H** ; **KLEINSZIG, G**. C-arm orbits for metal artifact avoidance (MAA) in cone-beam CT. *Physics in Medicine and Biology*, 2020, vol. 65 (16), 8, https://doi.org/10.1088/1361-6560/ab9454

- **ROHLEDER, M** ; **KUNZE, H.** ; **KLEINSZIG, G** ; **MAIER, A** ; **KREHER, B.** Robust 3D Metal Segmentation from X-Ray Projection Images for Metal Artifact Trajectory Optimization. *In: Medical Imaging 2024: Physics of Medical Imaging*, 2024, https://doi.org/10.1117/12.3005260
- **SIDDON, R.L.** Fast calculation of the exact radiological path for a three-dimensional CT array. *Medical Physics*, 1985, vol. 12 (2), 252-255, https://doi.org/10.1118/1.595715